# FASCICULE DE BREVET EUROPEEN

(11) **EP 1 592 414 B1**
(45) Date de publication et mention de la délivrance du brevet: **21.04.2010**
(21) Numéro de dépôt: 04711333.7
(22) Date de dépôt: 16.02.2004
(51) Int. Cl.: A61K 31/167, A61K 9/08

(54) **FORMULATION LIQUIDE INJECTABLE DE PARACETAMOL**
INJIZIERBARE FLÜSSIGE FORMULIERUNG VON PARACETAMOL
INJECTABLE LIQUID FORMULATION OF PARACETAMOL

(30) Priorité: 14.02.2003 FR 0301972
(43) Date de publication de la demande: 09.11.2005
(73) Titulaire: Nguyen-Xuan, Tho, 1267 Vich (CH)
(72) Inventeur: Nguyen-Xuan, Tho, 1267 Vich (CH)
(86) Numéro de dépôt international: PCT/CH2004/000085
(87) Numéro de publication internationale: WO 2004/071502

(56) Documents cités:
- EP-A- 0 916 347
- WO-A-98/05314
- WO-A-02/072080
- WO-A-03/033026
- POTTER D W ET AL: "IDENTIFICATION OF ACETAMINOPHEN POLYMERIZATION PRODUCTS CATALYZED BY HORSERADISH PEROXIDASE" JOURNAL OF BIOLOGICAL CHEMISTRY, vol. 260, no. 22, 1985, pages 12174-12180, XP002252373 ISSN: 0021-9258
- MASON R P ET AL: "FREE RADICALS OF ACETAMINOPHEN THEIR SUBSEQUENT REACTIONS AND TOXICOLOGICAL SIGNIFICANCE" FEDERATION PROCEEDINGS, vol. 45, no. 10, 1986, pages 2493-2499, XP008021010 ISSN: 0014-9446
- CLEGG, WILLIAM ET AL: "An oxidatively coupled dimer of paracetamol" ACTA CRYSTALLOGRAPHICA, SECTION C: CRYSTAL STRUCTURE COMMUNICATIONS (1998), C54(12), 1881-1882, XP008021024

## Description

La présente invention concerne une nouvelle formulation pharmaceutique liquide injectable de paracétamol contenant un dimère de ce principe actif, un procédé pour préparer cette formulation, et l'utilisation de ce dimère pour stabiliser une formulation pharmaceutique liquide de paracétamol.

Le paracétamol (DCI de l'acétaminophène ou N-(4-hydroxyphényl)acétamide) est un analgésique et un antipyrétique largement employé en milieu hospitalier. Il est souhaitable de disposer de formulations liquides pharmaceutiques stables de ce principe actif pour une administration par injection, en particulier pour perfusion par voie intraveineuse

Il est connu que le paracétamol en solution aqueuse est susceptible de subir une hydrolyse pour former du p-aminophénol, lui-même susceptible de se dégrader en quinoneimine (cf. par exemple J.E. Fairbrother, " Acetaminophen ", dans Analytical Profiles of Drug Substances, 1974, vol. 3, pp. 1-109). La vitesse de dégradation du paracétamol croît avec l'augmentation de la température et à la lumière. Cette vitesse est minimale à un pH voisin de 6 (K.T. Koshy et al., 1961, J. Pharm. Sci. 50, pp. 116-118).

Pour stabiliser le paracétamol en solution, il est connu d'ajouter un tampon et un agent antioxydant ou capteur de radicaux.

WO 02072080 par exemple décrit des solutions aqueuses pour perfusion stables de paracétamol comprenant un tampon de pH 5,5 à 6,5 et un agent antioxydant choisi parmi l'acide ascorbique et un dérivé porteur d'une fonction thiol tel que la cystéine ou l'acétyl-cystéine.

EP 0916347 divulgue des solutions de paracétamol à base d'un mélange d'eau et de solvants alcooliques comprenant un tampon de pH 5,5 à 5,6 et du métabisulfite comme agent antioxydant.

Les solutions injectables stabilisées de paracétamol de l'état de la technique présentent l'inconvénient de provoquer un effet potentiel irritant, allergisant et/ou cancérigène chez certains patients, à cause de la toxicité de l'agent antioxydant qu'elles contiennent. De plus leur stabilité nécessite l'élimination de l'oxygène et des autres oxydants du milieu aqueux. Ces solutions ne peuvent donc être conservées dans toxicité de l'agent antioxydant qu'elles contiennent. De plus leur stabilité nécessite l'élimination de l'oxygène et des autres oxydants du milieu aqueux. Ces solutions ne peuvent donc être conservées dans des récipients en matière plastique partiellement perméable à l'oxygène ou comportant des traces de résidus d'oxydants.

La demande de brevet MI2001A002135 (document de priorité de la demande WO 03/033026) non encore publiée divulgue des solutions aqueuses injectables stables de paracétamol obtenues par mélange de paracétamol avec de l'eau, du propylèneglycol et un tampon citrate (pH de 4,5 à 6,5), chauffage de la solution obtenue à une température comprise entre 70 et 130 °C et maintien de cette solution à cette température pendant au moins 10 minutes. Ces solutions ne contiennent pas d'agent antioxydant toxique, l'acide citrique étant un antioxydant faible largement utilisé dans l'industrie alimentaire.

D.W. Potter et al, 1985, J. Biol. Chem. 260, 22, pp.12174-80, divulguent l'oxydation et la polymérisation de paracétamol en solution aqueuse par le peroxyde d'hydrogène en présence de peroxydase de raifort, l'isolement par HPLC semi-préparative et l'identification par spectrométrie de masse et spectroscopie RMN, de deux dimères, deux trimères et deux tétramères de paracétamol. Pour une concentration de paracétamol supérieure à 0,2 mM, le dimère de formule (I) (" compound B ") est très majoritaire.

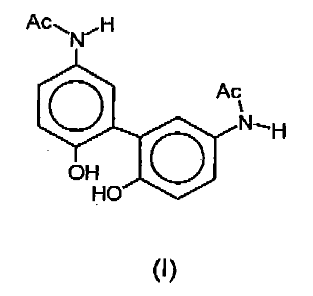

Le problème ou but de l'invention est de trouver une nouvelle formulation pharmaceutique liquide injectable de paracétamol qui soit stable pendant une durée prolongée sans présenter les inconvénients mentionnés ci-dessus.

Ce dimère de paracétamol même en très faible quantité semble agir comme antioxydant et permet de se passer des antioxydants forts ou toxiques décrits pour les solutions stabilisées de paracétamol de l'état de la technique.

La formulation de l'invention présente une excellente stabilité à température ambiante, et même à une température de l'ordre de 40 °C, et peut être conservée dans un récipient en matière plastique, en particulier une poche d'infusion, par exemple en polypropylène, polyéthylène, chlorure de polyvinyle ou en des combinaisons de polymères extrudés..

Ce dimère de paracétamol peut être remplacé par un autre produit de polymérisation du paracétamol, par exemple un mélange d'au moins deux oligomères de paracétamol choisis parmi les deux dimères, les deux trimères et les deux tétramères du paracétamol décrits par D.W. Potter et al., 1985, référence citée ci-dessus.

En général cette formulation contient au moins 0,005 %, de préférence au moins 0,05 %, en rapport de surface des pics HPLC avec une détection à 245 nm, de dimère de paracétamol de formule (I).

La formulation de l'invention peut contenir de 0,1 à 5,0 g/100ml, de préférence de 0,4 à 1,5 g / 100 ml de paracétamol.

Le solvant aqueux est de l'eau, de qualité pour injectable, ou un mélange d'eau et d'un ou plusieurs autre(s) solvant(s) miscible(s) à l'eau, par exemple le propylèneglycol, le polyéthylène glycol, l'éthanol, et/ou des surfactants tels que les polysorbates, les poloxamères. Si le taux de paracétamol souhaité dans la solution dépasse 1,0 g/100 ml, le solvant aqueux sera de préférence un mélange d'eau et de solvant(s) miscible(s) dans l'eau.

La formulation de l'invention contient un agent tamponnant de pKa compris entre 4,5 à 6,5, de préférence de 5,0 à 6,2. Cet agent tamponnant sera avantageusement choisi parmi le tampon citrate, le tampon phosphate, le tampon phosphate-citrate, le tampon bicarbonate, le tampon tartrate ou le tampon acétate, de préférence parmi le tampon citrate, le tampon phosphate et le tampon phosphate-citrate, ou un mélange de ces tampons.

Cette formulation pour injection contient un agent isotonisant, destiné à créer une pression osmotique voisine de celle du sérum physiologique. Cet agent isotonisant est choisi en général parmi le chlorure de sodium ou le glucose.

La formulation de l'invention est en général préparée d'abord par mélange de paracétamol, d'eau de qualité pour injectable, éventuellement d'un ou plusieurs autre(s) solvant(s) miscible(s) à l'eau, et/ou de surfactants, d'agent tamponnant et d'agent isotonisant, puis par chauffage de la solution obtenue, en vrac ou déjà remplie dans des récipients, à une température d'au moins 70 °C pendant au moins 15 minutes. Ce chauffage a pour but d'éliminer toute trace de nucléation qui pourrait lors du stockage de la solution déclencher une recristallisation du paracétamol.

L'invention concerne également un procédé de préparation de la formulation définie ci-dessus, qui comprend le mélange de paracétamol, d'eau, éventuellement de solvant miscible à l'eau, et/ou de surfactants d'agent tamponnant et d'agent isotonisant, puis le chauffage de la solution obtenue, en vrac ou déjà remplie dans des récipients, à une température d'au moins 70 °C pendant au moins 15 minutes

Le dimère de formule (I) semble se former spontanément mais lentement lors de la conservation de la solution obtenue ci-dessus à une température suffisante, par exemple de 60 °C.

Pour former in situ le dimère de formule (I), il est pratique de chauffer la solution à une température comprise entre 100 et 130 °C, de préférence entre 110 et 125 °C, pendant une durée d'au moins 5 minutes.

L'invention concerne aussi un procédé pour préparer la formulation définie plus haut qui comprend le chauffage de la solution à une température comprise entre 100 et 130 °C, de préférence entre 110 et 125 °C, pendant une durée d'au moins 5 minutes.

L'invention a aussi trait à une formulation telle que définie plus haut susceptible d'être obtenue par ce procédé.

L'invention concerne également l'utilisation du dimère de formule (I) pour stabiliser une formulation liquide de paracétamol. Ce dimère est en général fabriqué in situ au sein de la formulation liquide de paracétamol.

L'invention est exposée plus en détail dans les exemples ci-après, donnés à titre illustratif et non limitatif.

Dans ces exemples, la température est la température ambiante ou est exprimée en degré Celsius, et la pression est la pression atmosphérique. L'eau, le propylèneglycol et tous les réactifs utilisés sont de qualité pour injectable.

D'autre part, tous les exemples font partie intégrante de l'invention, ainsi que toute caractéristique de la description incluant les exemples, qui apparaît être nouvelle vis-à-vis d'un état de la technique quelconque, et ce sous forme de caractéristique générale et non pas de caractéristique particulière de l'exemple.

### Exemple 1 Préparation de formulations pharmaceutiques liquides selon l'invention et analyse de ces formulations par HPLC

Les formulations 001, 002, 003, 004, 005 et 006 ont été préparées, par mélange de paracétamol, d'eau ultrapurifiée de qualité pour injectable, éventuellement (formulation 004) de propylèneglycol, d'agent tamponnant (tampon phosphate, tampon citrate-phosphate ou tampon citrate) et d'agent isotonisant (chlorure de sodium), chauffage à 70-90 °C pendant environ 15 minutes pour éviter la possibilité de nucléation et par la suite de recristallisation du paracétamol, et remplissage de flacons en verre. Ces flacons ont ensuite été stérilisés pendant 15 minutes à 121 °C.

Les solutions ont été analysées, avant et après la stérilisation, par HPLC sur colonne de gel de silice octylsilyl et une phase mobile obtenue par mélange d'une solution de sel disodique d'hydrogénophosphate, d'une solution de sel sodique de dihydrogénophosphate, et de méthanol R contenant une solution de tétrabutylammonium R, et détection par spectrophotométrie à 245 nm, selon la méthode préconisée dans la Pharmacopée Européenne (European Pharmocopoeia 4.4, pp.3503-4, 04/2003 :0049 Paracétamol).

Un pic correspondant à une substance inconnue (non prévue par la Pharmacopée Européenne) été détecté. Cette substance a été identifiée par spectrométrie de masse couplée à une chromatographie liquide (LC-MS) et résonance magnétique nucléaire (RMN) du proton à 200 MHz, comme étant le dimère de paracétamol de formule (I), identique au composé B décrit par D.W. Potter et al, 1985, J. Biol. Chem. 260, 22, pp.12174-80.

La limite de détection est de l'ordre de 0,005 % pour le p-aminophénol et le dimère de formule (I). L'écart entre 100 et la valeur mesurée en paracétamol n'est pas significatif et représente une incertitude expérimentale.

Les compositions de ces formulations et les résultats de l'analyse HPLC sont rassemblés dans le Tableau 1 ci-après.

**Tableau 1**

| **Formulation** | 001 | 002 | 003 | 004¹⁾ | 005 | 006 |
|---|---|---|---|---|---|---|
| Paracétamol (g/100 ml) | 1,0 | 1,0 | 1,0 | 1,0 | 1,0 | 1,0 |
| Propylèneglycol (g/100 ml) | - | - | - | 0,8 | - | - |
| Phosphate monosodique dihydraté (g/100 ml) | 0,1002 | 0,1071 | 0,0467 | - | - | - |
| Phosphate disodique dihydraté (g/100 ml) | 0,0785 | - | 0,0899 | 0,0877 | - | 0,091 |
| Acide citrique monohydraté (g/100 ml) | - | - | - | 0,0499 | 0,033 | 0,045 |
| Citrate de sodium dihydraté | - | - | - | - | 0,131 | - |
| Chlorure de sodium (g/100 ml) | 0,65 | 0,65 | 0,7 | 0,6 | 0,65 | 0,675 |
| Eau pour injectable | ad 100ml | ad 100ml | ad 100ml | ad 100ml | ad 100ml | ad 100ml |
| pH | 5,7 | 4,7 | 6,9 | 5,2 | 5,3 | 5,4 |
| Osmolalité (mosm/kg) | 284 | 290 | 307 | 290 | 287 | 296 |

| **Analyse HPLC** | | | | | | |
|---|---|---|---|---|---|---|
| % Dimère (avant stérilisation)* | nd | nd | nd | nd | nd | nd |
| % Paracétamol (avant stérilisation) | 100,8 | 100,2 | 99,1 | 99,1 | 99,3 | 98,2 |
| % p-aminophénol (avant stérilisation) | nd | nd | nd | nd | nd | nd |
| % Dimère (après stérilisation)* | 0,07 | 0,02 | 0,12 | 0,04 | 0,05 | 0,08 |
| % Paracétamol (après stérilisation) | 100,7 | 100,7 | 98,5 | 98,6 | 99,1 | 98,6 |
| % p-aminophénol (après stérilisation) | nd | nd | nd | nd | nd | nd |

| | | | | | | |
|---|---|---|---|---|---|---|
| * rapport surface du pic du dimère/surface du pic du paracétamol en % nd : non détecté ¹⁾ : la formulation 004 n'est pas selon l'invention | | | | | | |

Pour ces formulations, un taux du dimère de formule (I) de 0,02 à 0,12 après la stérilisation a été mesuré, ce dimère n'étant pas détectable avant la stérilisation. La présence de p-aminophénol n'a pas été détectée avant ou après la stérilisation.

### Exemple 2 Etude de la formation du dimère

### 1) Influence de la température et de la durée

L'influence de la température sur la formation du dimère de formule (I) a été étudiée sur la formulation 001 de l'exemple 1 après son chauffage à 70-90 °C pendant 15 minutes et sa mise dans un flacon de verre. Cette formulation a été soumise à différentes températures de conservation, et/ou à différentes durées de stérilisation, et analysée par HPLC comme décrit ci-dessus dans l'Exemple 1.

Les principaux résultats sont rassemblés dans le Tableau 2 ci-dessous.

**Tableau 2 - Influence de la température et de la durée sur la formation du dimère**

| | Paracétamol (%) | p-aminophénol (%) | Dimère (%) |
|---|---|---|---|
| Conservé pendant 1 h à 70°C (sans stérilisation) | 100,8 | non détecté | non détecté |
| Conservé pendant 14 jours à 60°C (sans stérilisation) | 100,2 | non détecté | 0,10 |
| Stérilisé pendant 10 minutes à 121°C | 99,9 | non détecté | 0,09 |
| Stérilisé pendant 20 minutes à 121°C | 99,7 | non détecté | 0,18 |
| Conservé à 40°C pendant 14 jours, après stérilisation pendant 15 minutes à 121°C | 100,4 | non détecté | 0,4 |

Selon le tableau ci-dessus le dimère se forme rapidement à 121 °C, avec un taux de formation proportionnel à la durée de stérilisation, et lentement mais en quantité mesurable à 60 °C pendant 14 jours. Le dimère n'est pas détectable après 1 heure à 70 °C.

Dans toutes les conditions étudiées, le p-aminophénol n'est pas détecté.

### 2) Influence du pH

Les valeurs du taux de dimère de formule (I) reportées dans le Tableau 1 de l'Exemple 1 pour les formulations 001, 002 et 003 de compositions proches mais de pHs différents, montrent que la formation du dimère dépend du pH. Plus le pH est basique, plus la quantité de dimère formée est importante. Probablement la génération des radicaux est facilitée par la formation des phénolates.

### Exemple 3 Etude de la stabilité

La stabilité a été étudiée sur la formulation 001 de l'exemple 1 après le chauffage à 70-90 °C pendant 15 minutes, mise dans des flacons de verre ou des récipients pour perfusion en polypropylène, et stérilisation à 121 °C pendant 15 minutes pour les flacons en verre et à 120 °C pendant 20 minutes pour les récipients en polypropylène.

Le Tableau 3 ci-dessous rassemble les résultats obtenus par analyse HPLC comme décrit dans l'Exemple 1 ci-dessus..

**Tableau 3 : Stabilité de la formulation 001**

| | Flacon de verre 50ml | | | Récipient en polypropylène 100ml | | |
|---|---|---|---|---|---|---|
| | Départ | 10 mois | | Départ | 10mois | |
| | | 25°C | 40°C | | 25°C | 40°C |
| Osmolalité (mosm/kg) | 283 | 288 | 285 | 281 | 289 | 283 |
| Teneur en paracétamol (%) | 100,3 | 100,7 | 99,1 | 100,3 | 100,4 | 99,4 |
| Teneur en dimère (%) | 0,11 | 0,23 | 0,73 | 0,09 | 0,14 | 0,47 |
| Teneur en p-aminophénol (%) | nd | nd | nd | nd | nd | nd |
| pH | 5,6 | 5,6 | 5,6 | 5,6 | 5,6 | 5,6 |

| | | | | | | |
|---|---|---|---|---|---|---|
| nd : non détecté | | | | | | |

Ce tableau montre que la formulation liquide injectable selon l'invention conserve la même teneur en paracétamol après 10 mois de conservation à 25 °C ou 40 °C, avec une légère augmentation de la teneur en dimère, plus importante à 40 °C qu'à 25 °C, et dans les flacons de verre que dans les récipients en polypropylène.

La dégradation du paracétamol en solution est souvent étudiée par mesure des absorbances à 500 nm selon la méthode décrite par J .E. Fairbrother "Acetaminophen ", dans Analytical Profiles of Drug Substances, 1974, vol. 3, pp. 1-109). Le suivi pendant 3 semaines selon cette méthode de l'absorbance d'une part de la formulation 001 et d'autre part de la formulation commerciale Perfalgan® contenant 1,0 g/100ml de paracétamol et du chlorhydrate de cystéine, obtenue comme décrit dans WO9805314, montre qu'à 60°C le paracétamol se dégrade au moins 10 fois plus vite dans la formulation de Perfalgan® que dans la formulation de l'invention.

## Revendications

1. Formulation pharmaceutique liquide injectable de paracétamol qui contient du paracétamol, un solvant aqueux, un agent tamponnant de pKa compris entre 4,5 et 6,5, un agent isotonisant et du dimère de paracétamol de formule (I) suivante 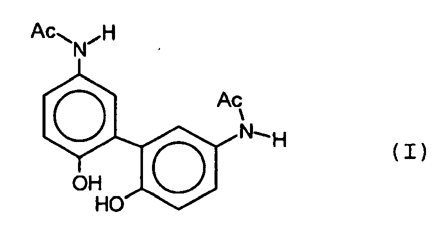 à l'exception d'une solution obtenue en mélangeant du paracétamol à l'eau, du propylèneglycol et un tampon citrate (pH de 4,5 à 6,5), en chauffant ladite solution de 70 °C à 130 °C et en la maintenant à ladite température pendant au moins 10 minutes.

2. Formulation selon la revendication 1, **caractérisée en ce qu'**elle contient au moins 0,005 %, en rapport de surface des pics HPLC avec une détection à 245 nm, de dimère de paracétamol de formule (I).

3. Formulation selon la revendication 1, **caractérisée en ce qu'**elle contient au moins 0,05 %, en rapport de surface des pics HPLC avec une détection à 245 nm, de dimère de paracétamol de formule (I).

4. Formulation selon l'une des revendications précédentes, **caractérisée en ce qu'**elle contient de 0,1 à 5,0 g/100ml de paracétamol.

5. Formulation selon l'une des revendications précédentes, **caractérisée en ce qu'**elle contient un agent tamponnant choisi parmi le tampon citrate, le tampon phosphate, le tampon phosphate-citrate, le tampon bicarbonate, le tampon tartrate ou le tampon acétate ou un mélange de ces tampons.

6. Formulation selon l'une des revendications précédentes, **caractérisée en ce qu'**elle est susceptible d'être obtenue par un procédé comprenant le chauffage de la solution à une température comprise entre 100 et 130 °C pendant une durée d'au moins 5 minutes.

7. Formulation selon l'une des revendications précédentes dans laquelle le dimère de formule (I) est remplacé par un autre produit de polymérisation du paracétamol.

8. Procédé de préparation d'une formulation selon l'une des revendications précédentes, qui comprend le mélange de paracétamol, d'eau, éventuellement d'un ou plusieurs solvant(s) miscible(s) à l'eau, et/ou de surfactants, d'agent tamponnant et d'agent isotonisant, et le chauffage de la solution à une température d'au moins 70 °C pendant au moins 15 minutes, puis à une température comprise entre 100 et 130 °C pendant une durée d'au moins 5 minutes.

9. Utilisation du dimère de formule (I) pour stabiliser une formulation liquide de paracétamol, qui n'est pas une solution obtenue en mélangeant du paracétamol à l'eau, du propylèneglycol et un tampon citrate (pH de 4,5 à 6,5), en chauffant ladite solution de 70 °C à 130 °C et en la maintenant à ladite température pendant au moins 10 minutes.

10. Utilisation d'un récipient en matière plastique, en particulier une poche d'infusion, pour conserver une formulation selon l'une des revendications 1 à 7.

## Claims

1. An injectable liquid pharmaceutical formulation of paracetamol, which contains paracetamol, an aqueous solvent, a buffer with a pKa of between 4.5 and 6.5, an isotonic agent and paracetamol dimer of formula (I) below : 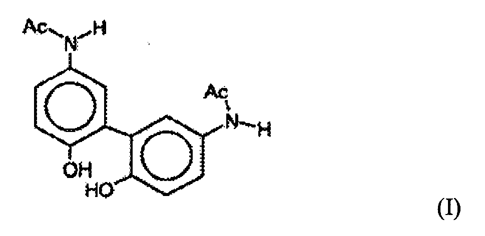 except for a solution obtained by mixing paracetamol with water, propylene glycol and a citrate buffer (pH between 4.5 to 6.5), by heating said solution from 70°C to 130°C and keeping same at said temperature for 10 minutes at least.

2. The formulation as claimed in claim 1, which contains at least 0.005%, as a ratio of the surface area of the HPLC peaks with detection at 245 nm , of paracetamol dimer of formula (I).

3. The formulation as claimed in claim 1, which contains at least 0.05%, as a ratio of the surface area of the HPLC peaks with detection at 245 nm , of paracetamol dimer of formula (I).

4. The formulation as claimed in one of the preceding claims, which contains from 0.1 to 5.0g/100ml of paracetamol.

5. The formulation as claimed in one of the preceding claims, which contains a buffer chosen from citrate buffer, phosphate buffer, phosphate-citrate buffer; bicarbonate buffer, tartrate buffer or acetate buffer or a mixture of these buffers.

6. The formulation as claimed in one of the preceding claims, which may be obtained via a process including heating of the solution at a temperature of between 100 and 130°C for a period of at least 5 minutes.

7. The formulation as claimed in one of the preceding claims, in which the dimer of formula (I) is replaced with another paracetamol polymerization product.

8. A process for preparing a formulation as claimed in one of the preceding claims, which includes mixing together paracetamol, water, optionally one or more water-miscible solvent(s), and/or surfactants, buffer and isotonic agent, and the heating of the solution at a temperature of at least 70°C for at least 15 minutes followed by a temperature of between 100 and 130°C for a period of at least 5 minutes.

9. The use of the dimer of formula (I) for stabilizing a liquid formulation of paracetamol, which is not a solution obtained by mixing paracetamol with water, propylene glycol and a citrate buffer (pH between 4.5 to 6.5), by heating said solution from 70°C to 130°C and keeping same at said temperature for 10 minutes at least.

10. The use of a plastic container, in particular an infusion bag, for containing a formulation as claimed in one of the claims 1 to 7.

## Patentansprüche

1. Injizierbare, pharmazeutische Paracetamol-Formulierung in flüssiger Form, welche Paracetamol, ein wässriges Lösungsmittel, ein Puffermittel mit pKa-Wert zwischen 4.5 und 6,5 eine isotonische Substanz und Paracetamol-Dimer mit folgender Formel (I) enthält 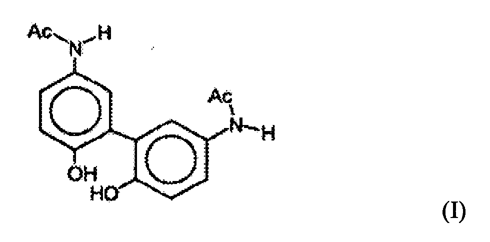 unter Ausschluss einer durch Mischen von Paracetamol mit Wasser, Propylenglykol und einem Zitratpuffer (pH zwischen 4,5 und 6,5) hergestellten Lösung, wobei die erwähnte Lösung auf eine Temperatur zwischen 70°C und 130°C erhitzt und mindestens 10 Minuten auf dieser Temperatur gehalten wird.

2. Formulierung nach Anspruch 1, **dadurch gekennzeichnet, dass** sie im Flächenverhältnis der HPLC-Spitzen mit einem Nachweis bei 245 nm mindestens 0,005 % Paracetamol-Dimer der Formel (I) enthält.

3. Formulierung nach Anspruch 1, **dadurch gekennzeichnet, dass** sie im Flächenverhältnis der HPLC-Spitzen mit einem Nachweis bei 245 nm mindestens 0,05 % Paracetamol-Dimer der Formel (I) enthält.

4. Formulierung nach einem der vorherigen Ansprüche, **dadurch gekennzeichnet, dass** sie zwischen 0,1 und 5,0 g/100ml Paracetamol enthält.

5. Formulierung nach einem der vorherigen Ansprüche, **dadurch gekennzeichnet, dass** sie ein Puffermittel enthält, das entweder ein Zitratpuffer, ein Phosphatpuffer, ein Phosphat-Zitratpuffer ein Bikarbonatpuffer ein Tartratpuffer oder Acetatpuffer oder ein Gemisch aus diesen Puffern ist.

6. Formulierung nach einem der vorherigen Ansprüche, **dadurch gekennzeichnet, dass** sie mit einem Verfahren hergestellt werden kann, bei dem die Lösung mindestens 5 Minuten auf eine Temperatur zwischen 100 und 130°C erhitzt wird.

7. Formulierung nach einem der vorherigen Ansprüche, in der das Formeldimer (I) durch ein anderes Polymerisationsprodukt des Paracetamols ersetzt wird.

8. Herstellungsverfahren für eine Formulierung nach einem der vorherigen Ansprüche, die ein Gemisch aus Paracetamol, Wasser, eventuell einem oder mehreren wasserlöslichen Lösungsmitteln und/oder grenzflächenaktiven Substanzen, Puffermitteln und eine isotonische Substanz enthält, wobei die Lösung mindestens 15 Minuten auf eine Temperatur von mindestens 70°C sowie anschließend für die Dauer von mindestens 5 Minuten auf eine Temperatur zwischen 100 und 130°C erhitzt wird.

9. Verwendung des Formeldimers (I) zur Stabilisierung einer flüssigen Paracetamol-Formulierung, die keine durch Mischen von Paracetamol mit Wasser, Propylenglykol und einem Zitratpuffer (pH zwischen 4,5 und 6,5) hergestellte Lösung ist, die auf eine Temperatur zwischen 70°C und 130°C erhitzt und mindestens 10 Minuten auf dieser Temperatur gehalten wird.

10. Verwendung eines Behälters aus Kunststoff, insbesondere einem Infusionsbeutel, zur Lagerung einer Formulierung nach einem der Ansprüche 1 bis 7.
